# EUROPÄISCHE PATENTSCHRIFT

(11) **EP 0 564 482 B1**
(45) Veröffentlichungstag und Bekanntmachung des Hinweises auf die Patenterteilung: **29.06.1994**
(21) Anmeldenummer: 92900397.8
(22) Anmeldetag: 07.12.1991
(51) Int. Cl.: D04H 5/06

(54) **ABDECKVLIES**
COVERING WEB
ETOFFE NAPPEE DE COUVERTURE

(30) Priorität: 27.12.1990 DE 4041999; 19.03.1991 DE 4108937
(43) Veröffentlichungstag der Anmeldung: 13.10.1993
(73) Patentinhaber: Corovin GmbH, D-31201 Peine (DE)
(72) Erfinder: FAHMY, Tarek, D-3150 Peine (DE)
(74) Vertreter: Thömen, Uwe, Dipl.-Ing.
(86) Internationale Anmeldenummer: DE9100962
(87) Internationale Veröffentlichungsnummer: WO9212281

(56) Entgegenhaltungen:
- EP-A- 0 403 840
- DE-A- 1 111 140
- DE-A- 2 356 720

## Beschreibung

Die Erfindung betrifft ein Abdeckvlies gemäß dem Oberbegriff des Patentanspruchs 1.

In der älteren Patentanmeldung P 39 20 066.3 ist ein als Adeckvlies verwendbares Verbundvliesmaterial beschrieben, welches sich durch ein Gemisch von groben Filamenten und feinen Mikrofasern auszeichnet, ohne daß dabei diskrete Schichten mit einer Phasengrenze zwischen den einzelnen Komponenten vorhanden sind. Die Bildung des Verbundvliesmaterials erfolgt vielmehr auf einer Legevorrichtung durch eine simultane Anblasung beider Komponenten, so daß das Verbundvliesmaterial aus einem Gemisch der groben Filamente und der feinen Mikrofasern besteht und somit ein integriertes Material darstellt.

Ein wesentlicher Vorteil dieses Verbundvliesmaterials besteht darin, daß wegen des gebildeten Gemisches der unterschiedlichen Komponenten ein Gradient über den Querschnitt im Hinblick auf die unterschiedlichen Faserdurchmesser nicht vorhanden ist. Gleichwohl liegt eine Zusammenfassung der beiden den unterschiedlichen Faserkomponenten anhaftenden Funktionen vor. Diese jeweiligen Funktionen erstrecken sich über den gesamten Querschnitt des Verbundvliesmaterials.

Da die einzelnen Komponenten über den gesamten Querschnitt gesehen miteinander vermischt sind, können die Komponenten auch über den gesamten Querschnitt die ihnen jeweils zugewiesenen Funktionen ausüben, was bei bekannten schichtförmigen Materialien mit diskreten Phasengrenzen nicht möglich ist. Eine denkbare Funktion ist bezüglich der feinen Mikrofasern die Filtration bzw. der Flüssigkeitstransport von Flüssigkeiten. Wegen der infolge der Durchmischung erreichten Verteilung der Mikrofasern über die Schichtstärke des Verbundvliesmaterials läßt sich eine höhere Filtrationsgeschwindigkeit erzielen.

Insgesamt beschreibt also die ältere Patentanmeldung ein verbessertes Verbundvliesmaterial, welches hinsichtlich der den einzelnen Komponenten anhaftenden Funktionen eine erhöhte Effizienz besitzt. Dadurch erschließen sich dem Verbundvliesmaterial wesentlich verbesserte Einsatzmöglichkeiten.

Es gibt allerdings gleichwohl noch Anwendungsgebiete, in denen sich auch das genannte Verbundvliesmaterial nicht optimal verwenden läßt. Der Erfindung liegt daher die Aufgabe zugrunde, das genannte Verbundvliesmaterial in der Weise auszugestalten, daß das Einsatzgebiet noch vielfältiger und wirkungsvoller wird, insbesondere bei der Verwendung des Verbundvliesmaterials als Abdeckvlies für den Saugkörper von saugfähigen Produkten, wie Windeln, Binden oder dergleichen.

Die Lösung dieser Aufgabe erfolgt bei dem im Oberbegriff des Patentanspruchs 1 genannten Abdecklies durch die Merkmale des kennzeichnenden Teils.

Der grundlegende Gedanke der Erfindung besteht darin, das Verbundvliesmaterial so auszubilden, daß es den jeweiligen Anforderungen entsprechende unterschiedliche Bereiche aufweist, in denen unterschiedliche Mischungsverhältnisse der beiden Komponenten (grobe Filamente und feine Mikrofasern) vorliegen. Dadurch lassen sich nämlich voneinander getrennte Bereiche des Verbundvliesmaterials realisieren, die unterschiedliche Funktionen übernehmen können.

Ein solches Vliesmaterial bildet beispielsweise bei Windeln einen wesentlichen Vorteil, wenn das Verbundvliesmaterial als Abdeckschicht bzw. Abdeckvlies für den Saugkörper der Windel verwendet wird. Durch die unterschiedlichen Mischungsverhältnisse lassen sich nämlich im Hinblick auf die Feuchtigkeitsdurchlässigkeit bzw. auf die feuchtigkeitsabweisende Funktion gewünschte unterschiedliche Bereiche der Windeloberfläche realisieren.

Dabei ist es möglich, eine Hydrophilierung nur im mittleren Teil der Abdeckschicht der Windel vorzusehen und hier also den eigentlichen Drainagebereich auszubilden. Die beiden seitlichen Streifen können vorteilhaft hydrophob ausgebildet werden.

Bei der Erfindung ist z. B. vorgesehen, daß das Verbundvliesmaterial nur teilweise aus zwei Komponenten besteht, so daß ein oder mehrere diskrete Bereiche vorgesehen sind, die ausschließlich durch die groben Filamente gebildet sind. Bei dieser Ausführungsform gibt es also diskrete Bereiche, die kein Gemisch von groben und feinen Filamenten darstellen.

Vorzugsweise sind die genannten diskreten Bereiche durch parallel zueinander und im Abstand voneinander verlaufende Streifen gebildet. Dadurch ergibt sich ein bevorzugtes Anwendungsgebiet bei der Verwendung des Verbundvliesmaterials als Abdeckschicht bzw. Abdeckvlies für eine Windel, Damenbinden, Inkontinenzprodukten, für den gesamen Hygienebereich usw..

Anhand der in der Zeichnung dargestellten Ausführungsbeispiele wird die Erfindung nachfolgend näher erläutert. In der Zeichnung zeigen:
- Fig. 1: eine schematische Querschnittsansicht eines älteren Verbundvliesmaterials ,
- Fig. 2: eine vergrößerte und vereinfachte Querschnittsdarstellung des Verbundvliesmaterials gemäß Fig. 1,
- Fig. 3: eine Draufsicht auf ein Verbundvliesmaterial gemäß der ersten Variante der Erfindung,
- Fig. 4: eine schematische Querschnittsansicht des Verbundvliesmaterials gemäß Fig. 3,
- Fig. 5: eine schematische Querschnittsansicht der zweiten Variante der Erfindung,
- Fig. 6: eine schematische Querschnittsansicht einer Ausführungsform der Erfindung, und
- Fig. 7: eine schematische Querschnittsansicht einer Windel unter Verwendung eines Verbundvliesmaterials als obere Abdeckschicht.

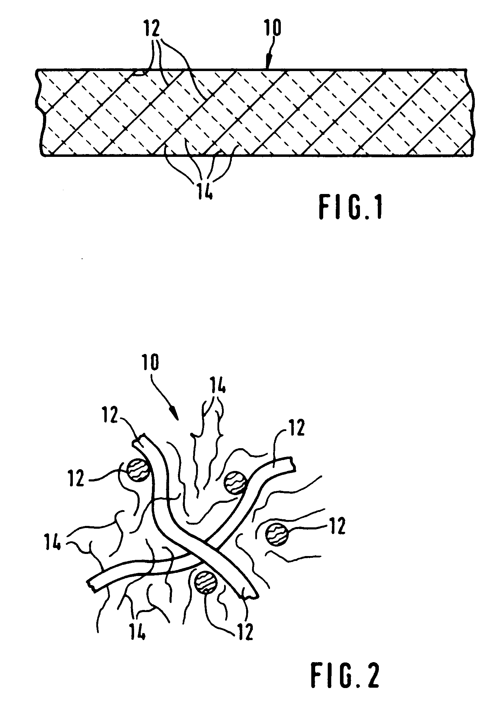

Anhand von Fig. 1 und 2 wird zunächst ein Verbundvliesmaterial 10 gemäß der eingangs genannten Patentanmeldung P 39 20 066.3 beschrieben, von der die Erfindung ausgeht. Das Verbundvliesmaterial 10 besteht aus einem Gemisch von groben Filamenten 12 und feinen Mikrofasern 14.

Zur Verdeutlichung, daß das Verbundvliesmaterial 10 keine diskreten Schichten mit einer Phasengrenze besitzt, sondern ein Gemisch darstellt, sind die groben Filamente 12 in Fig. 1 mit durchgezogenen Schraffurlinien und die feinen Mikrofasern 14 durch gestrichelt gezeichnet Schraffurlinien angedeutet. Sowohl die groben molekularorientierten und im wesentlichen endlosen Filamente 12 als auch die im wesentlichen kaum molekularorientierten diskontinuierlichen feinen Mikrofasern 14 erstrecken sich im wesentlichen über die gesamte Stärke des Querschnittes des Verbundvliesmaterials 10. Alternativ zu den im wesentlichen kaum molekularorientierten diskontinuierlichen feinen Mikrofasern 14 können auch feine Endlos-Mikrofilamente beliebiger Molekularorientierung zum Einsatz kommen.

Die Herstellung des Verbundvliesmaterials 10 erfolgt in einem integrierten Vliebildungsvorgang auf der gleichen Legevorrichtung einer nicht weiter dargestellten Vliesspinnanlage. Die Filamente 12 und die Mikrofasern 14 werden dabei zu einem Flächengebilde abgelegt, ohne daß schichtförmige diskrete Phasengrenzen entstehen.

Wie die stark vergrößerte und vereinfachte Darstellung in Fig. 2 verdeutlicht, sind die Filamente 12 und die Mikrofasern 14 miteinander vermengt, wodurch das Gemisch entsteht. Dabei füllen die meist kurzen und sehr feinen Mikrofasern 14 weitgehend die Zwischenräume zwischen den vergleichsweise groben Filamenten 12 aus, wodurch das Verbundvliesmaterial schon eine gewisse Verfestigung erhält. Das Gemisch des Verbundvliesmaterials 10 wird im übrigen gebildet, ohne daß die einzelnen Komponenten, also die Filamente 12 oder die Mikrofasern 14, vorher eine Zwischenverfestigung erfahren haben.

Die Durchmesser der groben Filamente 12 liegen in einer Größenordnung von mehr als 15 um, während die Durchmesser der wesentlichen feineren Mikrofasern 14 Werte von kleiner als 10 um aufweisen.

Bei den endlosen molekularorientierten Filamenten 12, welche eine tragende Matrix des Verbundvliesmaterials 10 bilden, kann es sich um ein übliches Spinnvliesmaterial handeln. Die im wesentlichen diskontinuierlichen Mikrofasern 14 lassen sich in vorteilhafter Weise nach z.B. dem Melt-Blown-Verfahren herstellen. Diese feinen Mikrofasern werden mit hoher Geschwindigkeit auf die unmittelbar zuvor abgelegte Schicht aus groben Filamenten aufgebracht, so daß sie in Hohlräume dieser groben Schicht eindringen.

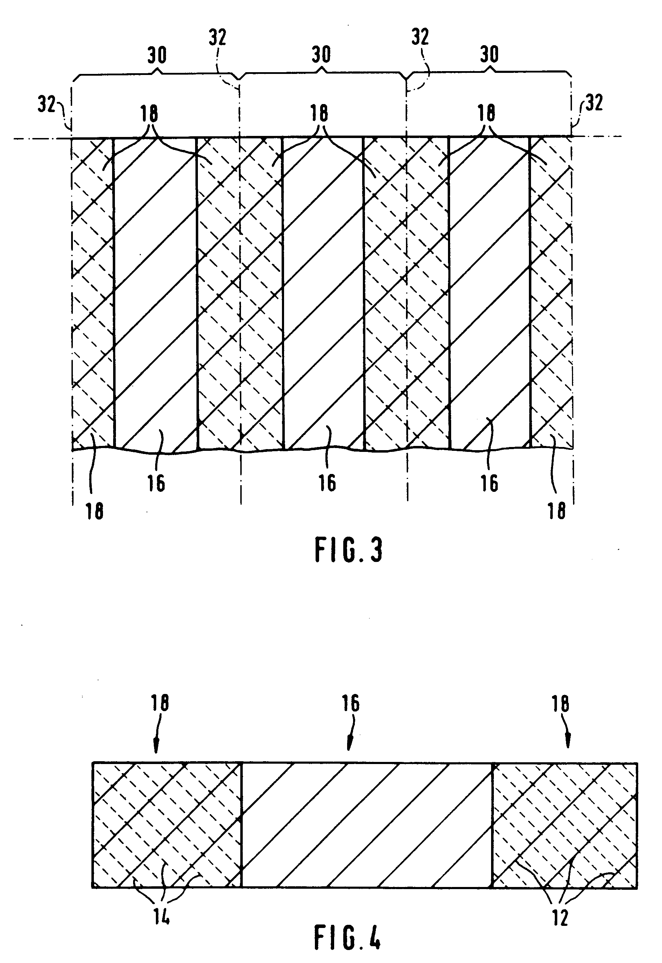

Fig. 3 und 4 zeigen eine erste Variante der Erfindung.

Gemäß der Draufsicht auf eine Bahn gemäß Fig. 3 umfaßt das Verbundvliesmaterial abwechselnd diskrete streifenförmige Bereiche 16 und 18. Die streifenförmigen Bereiche 16 bestehen ausschließlich aus groben Filamenten, sind also in normaler Weise als Spinnvlies ausgebildet.

Demgegenüber besteht das Verbundvliesmaterial im Bereich der streifenförmigen Bereiche 18 aus einem Gemisch der beiden Komponenten, nämlich aus groben Filamenten 12 und feinen Mikrofasern 14. Durch die gestrichelt gezeichneten Linien 32 ist in Fig. 3 angedeutet, daß sich aus der dargestellten Bahn durch Zerschneiden längs der genannten Linien 32 Windelbahnen 30 bilden lassen. Jede Windelbahn 30 besitzt dann als Abdeckung zwei äußere Streifen 18, und zwischen diesen beiden äußeren Streifen 18 befindet sich der diskrete Bereich 16, der ausschließlich durch Spinnvlies gebildet ist.

Im ursprünglichen Zustand ist die gesamte Windelbahn 30 zunächst hydrophob. Für die Verwendung der Windelbahn 30 bei einer Windel wird nur der mittlere Streifen 16 hydrophiliert, so daß dieser mittlere Streifen 16 einen Drainagebereich bildet. Die beiden äußeren Streifen 18 sind hydrophob und dienen zur Abdeckung der seitlichen Nebenzonen eines hier nicht näher dargestellten Saugkörpers einer Windel.

Zur näheren Verdeutlichung zeigt Fig. 4 eine Querschnittsansicht einer Windelbahn 30. An den beiden äußeren Seiten sind die streifenförmigen Bereiche 18 zu erkennen, die sowohl durch die groben Filamente als auch durch die feinen Mikrofasern gebildet sind. Dazwischen befindet sich der ausschließlich aus den groben Filamenten bestehende Bereiche 16.

Der dargestellte Aufbau einer Windelbahn 30 ermöglicht es, im Bereich der beiden äußeren Streifen 18 die besonders ausgeprägte hydrophobe Eigenschaft der feinen Mikrofasern (Melt-Blown) auszunutzen, so daß bei einer Windel, dessen Saugkörper mit einer Windelbahn 30 abgedeckt ist, eine Rückwässerung von Flüssigkeit des Saugkörpers in den seitlichen Nebenzonen sicher vermieden wird. Im mittleren Drainagebereich - Streifen 16 - kann demgegenüber Flüssigkeit zum Saugkörper gelangen. Wenn in diesem Bereich auch eine gewisse Rückwässerung möglich sein könnte, so bleibt als Vorteil, daß eine solche Rückwässerung eben nur auf dem mittleren streifenförmigen Bereich 16 beschränkt ist und nicht in den Nebenzonen des Saugkörpers, also im Bereich der äußeren Streifen 18 auftritt.

Dies ist ein wesentlicher Vorteil gegenüber herkömmlichen Windeln, bei denen die gesamte Abdeckschicht des Saugkörpers vollflächig hydrophil ausgebildet ist.

Wird eine Windelbahn gemäß Fig. 4 quer zur Produktionsrichtung der Windel eingesetzt, so kommen die äußeren, hydrophoben Streifen 18 an den Vorder- und Hinterenden der Windel zu liegen. Beim Tragen der Windel ergibt sich somit eine geringere Rücknässung im Bauch- bzw. Rückenbereich.

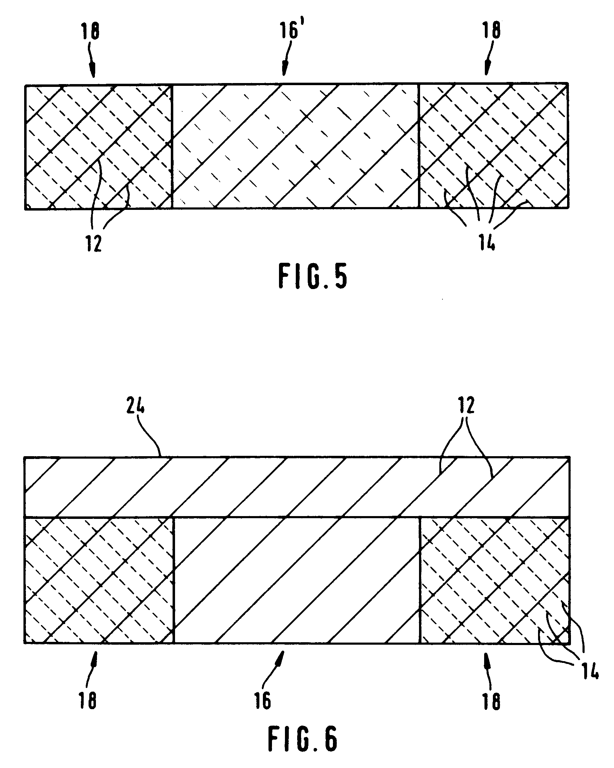

Die andere Variante der Erfindung gemäß Fig. 5 unterscheidet sich von der Darstellung gemäß Fig. 4 dadurch, daß der mittlere Streifen 16′ nicht ausschließlich durch grobe Filamente 12 gebildet wird, sondern auch einen gewissen Anteil der feinen Mikrofasern 14 enthält. Jedoch ist dieser Anteil gering im Vergleich zu dem Anteil der feinen Mikrofasern, die in den beiden äußeren Randstreifen 18 vorgesehen werden.

Ausgehend von dem ursprünglichen hydrophoben Zustand der gesamten Oberfläche wird auch hier wieder lediglich der mittlere Bereich 16′ einer Hydrophilierung unterzogen, um einen Drainagebereich zu schaffen. Die beiden äußeren Streifen 18 bleiben also hydrophob, um die Nebenzonen eines Saugkörpers abzudecken und trocken zu halten.

Der Vorteil der Variante gemäß Fig. 5 besteht darin, daß die Windelbahn 30 im Drainagebereich 16′ zwar hydrophil ist, daß jedoch wegen des geringen Anteils an feinen Mikrofasern 14 eine Penetrierung von kleinen Partikeln vermieden werden kann. Solche kleinen Partikel befinden sich beispielsweise in dem Saugkörper als superabsorbierendes Pulver, welches bei der Herstellung von Saugkörpern verwendet wird.

Die Verhinderung einer möglichen Penetrierung dieser kleinen Partikel gewährleistet also, daß diese durch den mittleren Streifen 16′ hindurch nicht nach außen gelangen können.

Eine andere Realisierung der Erfindung ist in Fig. 6 dargestellt und wird in der Weise gebildet, daß auf die eine Seite der Windelbahn 30 gemäß Fig. 4 zusätzlich noch eine Vliesmaterialschicht 24 aufgebracht wird, die ausschließlich aus groben Filamenten besteht, also ein übliches Spinnvlies darstellt (diese Vliesmaterialschicht kann auch auf die Windelbahn gemäß Fig. 5 aufgebracht werden).

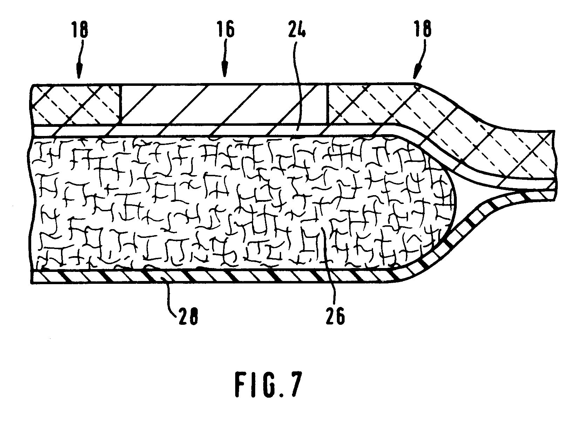

Der Zweck der zusätzlichen Vliesmaterialschicht 24 wird anhand von Fig. 7 deutlich, die in schematischer Querschnittsansicht einen Teil einer Windel zeigt. Oben ist der Saugkörper 26 der Windel mit dem Verbundvliesmaterial gemäß Fig. 6 abgedeckt, welches somit die Abdeckschicht bildet. Dabei kommt die in Fig. 6 oben liegende Seite hier nach unten, also in Richtung des Saugkörpers 26, zu liegen.

An der unteren Seite des Saugkörpers 26 befindet sich in bekannter Weise eine durch eine Folie gebildete Außenhaut 28 (Back-Sheet-Folie). Diese Außenhaut muß seitlich außerhalb des Saugkörpers 26 mit der Abdeckschicht verbunden und befestigt werden, so daß der Saugkörper 26 vollständig eingeschlossen ist. Die Befestigung erfolgt üblicherweise durch Verkleben der Abdeckschicht mit der Außenhaut an den seitlichen Rändern der Windel.

In der Praxis hat sich gezeigt, daß diese Verklebung mangelhaft sein kann, wenn die obere Abdeckschicht feine Mikrofasern enthält. Um diesem möglichen Nachteil zu begegnen, wird die zusätzliche Vliesmaterialschicht 24 eingesetzt, die aus Spinnvlies besteht und einen grobfaserigen Aufbau besitzt. Dadurch wird es möglich, die obere Abdeckschicht sicher mit der Außenhaut 28 zu verkleben, weil der Kleber besser in das Fasergebilde eindringen kann.

Im Falle von Damenbinden kann eine Warenbahn gemäß Fig. 4, 5, 6 so um einen Saugkörper gefaltet werden, daß die hydrophoben Mikrofasern enthaltenden Seitenstreifen 18 auf die Unterseite der Damenbinde zu liegen kommen. Der hydrophile (vorwiegend) aus Spinnvlies bestehende Mittelstreifen stellt dann die feuchtigkeitsdurchläsige Oberseite der Binde dar, während die hydrophoben Streifen den seitlichen und rückseitigen Auslaufschutz bilden.

## Patentansprüche

1. Abdeckvlies für saugfähige Produkte, wie Windeln, Binden oder dergleichen, bestehend aus einem Verbundvliesmaterial aus wenigstens zwei Komponenten, nämlich aus im wesentlichen endlosen, molekularorientierten groben Filamenten mit relativ großen Durchmessern, und aus im wesentlichen kaum molekularorientierten diskontinuierlichen feinen Mikrofasern oder aus feinen Endlos-Mikrofasern beliebiger Molekularorientierung mit relativ kleinen Durchmessern, wobei das Verbundvliesmaterial durch ein Gemisch der Komponenten (grobe Filamente und feine Mikrofasern) ohne diskrete schichtförmige Phasengrenzen zwischen den Komponenten gebildet ist, und wobei das Verbundvliesmaterial in einem integrierten Vliesbildungsvorgang auf ein und derselben Legevorrichtung einer Vliesspinnanlage hergestellt ist, **dadurch gekennzeichnet**, daß das Mischungsverhältnis der beiden Komponenten (12, 14) (grobe Filamente und feine Mikrofasern) an unterschiedlichen Stellen (16, 18) des Verbundvliesmaterials (12) in der Weise verschiedenartig gewählt ist, daß das Verbundvliesmaterial (12) diskrete Bereiche (16) aufweist, in denen der Anteil der feinen Mikrofasern (14) im Vergleich zu dem Anteil der feinen Mikrofasern (14) in den anderen Bereichen (18) deutlich kleiner ist, oder in der Weise, daß das Verbundvliesmaterial (12) nicht vollständig, sondern nur teilweise aus zwei Komponenten (12, 14) besteht, so daß ein oder mehrere diskrete Bereiche (16) vorgesehen sind, die ausschließlich durch die groben Filamente (12) gebildet sind.

2. Verbundvliesmaterial nach Anspruch ,**dadurch** **gekennzeichnet****,** daß die diskreten Bereiche (16) durch mehrere parallel zueinander und im Abstand voneinander verlaufende Streifen gebildet sind.

3. Verbundvliesmaterial nach einem der vorhergehenden Ansprüche 1 und/oder 2, **dadurch gekennzeichnet****,** daß auf einer Seite des Verbundvliesmaterials (12) eine nur aus groben Filamenten bestehende Vliesmaterialschicht (24) aufgebracht ist.

## Claims

1. Covering web for absorbent products, such as nappies, bandages or the like, consisting of a composite non-woven material of at least two components, namely of essentially endless, molecularly orientated coarse filaments of relatively large diameters, and of essentially scarcely molecularly orientated, discontinuous fine microfibres or of fine endless microfibres of any molecular orientation of relatively small diameters, the composite non-woven material being formed by a mixture of the components (coarse filaments and fine microfibres) without discrete layer-shaped phase boundaries between the components, and the composite non-woven material being made in an integrated non-woven forming operation on one and the same laying apparatus of a tissue spinning installation, characterized in that the mixture ratio of the two components (12, 14) (coarse filaments and fine microfibres) is chosen to vary at different positions (16, 18) of the composite non-woven material (12) in such a way that the composite non-woven material (12) possesses discrete regions (16) in which the proportion of the fine microfibres (14) compared to the proportion of the fine microfibres (14) in the other regions (18) is definitely smaller, or in such a way that the composite non-woven material (12) consists not entirely but only partly of two components (12, 14), so that one or more discrete regions (16) are provided, which are formed exclusively of the coarse filaments (12).

2. Composite non-woven material according to Claim 1, characterized in that the discrete regions (16) are formed of a plurality of mutually parallel strips, spaced apart from one another.

3. Composite non-woven material according to one of the preceding Claims 1 and/or 2, characterized in that, on one side of the composite non-woven material (12), a non-woven material layer (24) consisting only of coarse filaments is applied.

## Revendications

1. Etoffe nappée de recouvrement pour produits absorbants, comme des langes, des bandages, etc., se composant d'une matière composite en non tissé d'au moins deux composants, à savoir de gros filaments pratiquement continus à orientation moléculaire de diamètre relativement grand et en microfibres fines discontinues pratiquement peu orientées moléculairement ou en fines microfibres continues à orientation moléculaire quelconque d'un diamètre relativement petit, la matière composite en non tissé étant constituée d'un mélange de composants (gros filaments et fines microfibres) sans frontières de phase sous forme de couches discrètes entre les composants, et la matière composite en non tissé étant fabriquée par un procédé intégré de fabrication de non tissés sur un même appareil de pose d'une installation de filage de non tissé caractérisée en ce que la proportion du mélange des deux composants (12, 14) (gros filaments et fines microfibres) est choisie de façon distincte en différents endroits (16, 18) de la matière composite en non tissé (12), en ce que la matière composite en non tissé (12) présente des zones discrètes (16) dans lesquelles la proportion des microfibres (14) est clairement plus petite par rapport à celle des fines microfibres (14) dans les autres zones (18), ou de façon que la matière composite en non tissé (12) ne consiste pas complètement mais seulement partiellement de deux composants (12, 14) de manière que soient prévues une ou plusieurs zones discrètes (16) qui sont faites exclusivement de gros filaments (12).

2. Matière composite en non tissé suivant la revendication 1, caractérisée en ce que les zones discrètes (16) sont constituées par plusieurs bandes parallèles et séparées.

3. Matière composite en non tissé suivant l'une des revendications précédentes 1 et/ou 2, caractérisée en ce que, sur un côté de la matière composite en non tissé (12), est ajoutée une couche en matière en non tissé (24) faite uniquement en gros filaments.
